# EUROPÄISCHE PATENTANMELDUNG

(11) **EP 2 591 767 A2**
(43) Veröffentlichungstag der Anmeldung: **15.05.2013**
(21) Anmeldenummer: 12180530.3
(22) Anmeldetag: 15.08.2012
(51) Int. Cl.: A61K 8/73, A61K 8/81, A61Q 19/06

(54) **Hautstraffende kosmetische Zusammensetzungen mit angenehmer Produkthaptik**

(30) Priorität: 23.08.2011 DE 102011081434
(71) Anmelder: Henkel AG & Co. KGaA, 40589 Düsseldorf (DE)
(72) Erfinder: Welß, Thomas, 40591 Düsseldorf (DE); Emond, Eliane, 69003 Lyon (FR)

(57) **Zusammenfassung**

Kosmetische oder dermatologische Zusammensetzungen zur topischen Behandlung der Haut, die in einem geeigneten kosmetischen oder dermatologischen Träger - jeweils bezogen auf ihr Gewicht - 0,01 bis 5 Gew.-% Xanthan, 0,01 bis 5 Gew.-% Dehydroxanthan und 0,1 bis 10 Gew.-% Polystyrolsulfonat(e). enthalten, führen zu Sofortstraffungseffekten, wobei der oft negative Einfluß natürlicher Polymere auf die Produktkonsistenz kompensiert ist.

## Beschreibung

Die vorliegende Erfindung betrifft topisch zu applizierende kosmetische Zusammensetzungen zur Straffung und Glättung der Haut.

Das Körperbewusstsein der Verbraucher ist in den vergangenen Jahren deutlich gestiegen, was der Anstieg des Verbrauchs von kosmetischen Zusammensetzungen zeigt. Dabei werden neben Zusammensetzungen zur reinigenden und pflegenden Anwendung zunehmend auch kosmetische Zusammensetzungen nachgefragt, die helfen, die Körpersilhouette zu verbessern.

Mittel und Behandlungsmethoden zur Straffung und Glättung der Haut sind dabei eine bedeutende kosmetische Herausforderung. Bei diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Neben der nachhaltigen Wirkung, die im Idealfall sofort wahrnehmbar eintritt, erwartet der Verbraucher auch ein ästhetisch optimiertes Produkt. Kosmetika zur Straffung und Glättung der Haut haben oft den Nachteil, daß sie dem Verbraucher zäh und klebrig erscheinen, was in vielen Fällen durch ein Fadenziehen bei der Entnahme des Produktes nicht nur haptisch, sondern auch visuell wahrnehmbar ist.

Eine Aufgabe der vorliegenden Erfindung war es, die Nachteile des Standes der Technik zu beseitigen. Insbesondere war es eine Aufgabe der vorliegenden Erfindung, kosmetische Zusammensetzungen zur Verfügung zu stellen, die eine angenehme Produkthaptik aufweisen, bei der Entnahme und Applikation keine Fäden ziehen und eine sofortige Straffung der Haut ermöglichen.

Es wurde nun gefunden, daß sich Sofortstraffungseffekte durch bestimmte natürliche Polymere realisieren lassen, wobei der oft negative Einfluß dieser Verbindungen auf die Produktkonsistenz durch die Zugabe bestimmter synthetischer Polymere kompensiert werden kann.

Gegenstand der vorliegenden Erfindung sind in einer ersten Ausführungsform kosmetische oder dermatologische Zusammensetzungen zur topischen Behandlung der Haut, enthaltend in einem geeigneten kosmetischen oder dermatologischen Träger - jeweils bezogen auf ihr Gewicht -
a) 0,01 bis 5 Gew.-% Xanthan,
b) 0,01 bis 5 Gew.-% Dehydroxanthan,
c) 0,1 bis 10 Gew.-% Polystyrolsulfonat(e).

Erfindungsgemäß besonders bevorzugte Zusammensetzungen sind **dadurch gekennzeichnet, daß** sie 0,05 bis 4 Gew.-%, vorzugsweise 0,1 bis 3 Gew.-%, weiter bevorzugt 0,2 bis 2 Gew.-%, noch weiter bevorzugt 0,25 bis 1,5 Gew.-% und insbesondere 0,3 bis 1 Gew.-% Xanthan enthalten.

Xanthan ist ein natürliches Polymer, das mit Hilfe von Bakterien der Gattung Xanthomonas aus zuckerhaltigen Substraten gewonnen wird. Das Rückgrat des Xanthan-Polymers wird von β-(1→4)-verknüpften D-Glucoseeinheiten gebildet. An jede zweite Glucoseeinheit ist α-(1→3)-glycosidisch eine β-D-Mannopyranosyl-(1→4)-β-D-glucuronopyranosyl-(1→2)-6-O-acetyl-α-D-mannopyranosyl-Seitenkette geknüpft. Etwa die Hälfte der endständigen Mannoseeinheiten dieser Seitenkette bildet über die Hydroxylgruppen an den Positionen 4 und 6 ein Ketal mit Brenztraubensäure. Gelegentlich kann die Acetylgruppe oder eine Seitenkette komplett fehlen.

Als zweiten Bestandteil enthalten die erfindungsgemäßen Zusammensetzungen Dehydroxanthan Gum (dehydratisiertes Xanthan-Gum mit der INCI-Bezeichnung Dehydroxanthan Gum). Die Herstellung von dehydratisiertem Xanthan-Gum ist z.B. aus JP 07278202 A bekannt. Dehydratisiertes Xanthan-Gum mit der INCI-Bezeichnung Dehydroxanthan Gum ist unter dem Handelsnamen Amaze ^{®}XT von National Starch/Akzo Nobel erhältlich.

Auch Dehydroxanthan wird in den erfindungsgemäßen Zusammensetzungen vorzugsweise innerhalb engerer Mengenbereiche eingesetzt. Hier sind bevorzugte erfindungsgemäße Zusammensetzungen **dadurch gekennzeichnet, daß** sie 0,05 bis 4 Gew.-%, vorzugsweise 0,1 bis 3 Gew.-%, weiter bevorzugt 0,2 bis 2 Gew.-%, noch weiter bevorzugt 0,25 bis 1,5 Gew.-% und insbesondere 0,4 bis 1 Gew.-% Dehydroxanthan enthalten.

Es ist erfindungsgemäß bevorzugt, Xanthan und Dehydroxanthan in einem Gewichtsverhältnis von 1:1 oder darunter einzusetzen, d.h. entweder gleiche Mengen an Xanthan und Dehydroxanthan oder einen Überschuß an Dehydroxanthan. In bevorzugten erfindungsgemäßen Zusammensetzungen beträgt das Gewichtsverhältnis von Xanthan zu Dehydroxanthan 1:1 bis 5:10, vorzugsweise 1:1 bis 6:10, weiter bevorzugt 1:1 bis 7:10 und insbesondere 8:10 bis 9:10.

Als dritten wesentlichen Bestandteil enthalten die erfindungsgemäßen Zusammensetzungen 0,1 bis 10 Gew.-% Polystyrolsulfonat(e). Besonders bevorzugte erfindungsgemäße Mittel sind **dadurch gekennzeichnet, daß** sie 0,25 bis 7,5 Gew.-%, vorzugsweise 0,5 bis 5 Gew.-%, weiter bevorzugt 0,75 bis 4 Gew.-%, noch weiter bevorzugt 1 bis 3 Gew.-% und insbesondere 1,5 bis 2,5 Gew.-% Polystyrolsulfonat(e) enthalten.

Weiter bevorzugte erfindungsgemäße Mittel sind **dadurch gekennzeichnet, daß** sie Natrium-Polystyrolsulfonat(e) mit Struktureinheiten der Formel (PS) enthalten 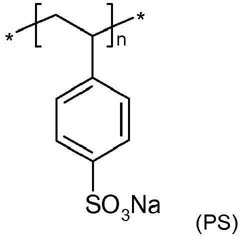 in der n für Werte von 100 bis 1500, vorzugsweise von 200 bis 1250, weiter bevorzugt von 300 bis 1000 und insbesondere von 400 bis 800 steht.

Besonders bevorzugte erfindungsgemäße Mittel sind **dadurch gekennzeichnet, daß** die Polymere b1 Molmassen von 10 bis 400 kDa, vorzugsweise von 25 bis 300 kDa, weiter bevorzugt von 50 bis 200 kDa und insbesondere von 100 bis 150 kDa, aufweisen.

Zusammenfassend sind insbesondere erfindungsgemäße Zusammensetzungen bevorzugt, die 0,25 bis 7,5 Gew.-%, vorzugsweise 0,5 bis 5 Gew.-%, weiter bevorzugt 0,75 bis 4 Gew.-%, noch weiter bevorzugt 1 bis 3 Gew.-% und insbesondere 1,5 bis 2,5 Gew.-% Natrium-Polystyrolsulfonat 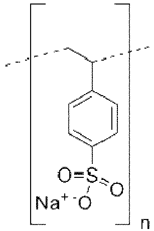 mit n = 100 bis 1000, vorzugsweise 200 bis 900, weiter bevorzugt 300 bis 800, noch weiter bevorzugt 400 bis 750 und insbesondere 600 bis 700, enthalten.

In einer weiteren bevorzugten Ausführungsform enthalten die erfindungsgemäßen Zusammensetzungen mindestens ein Polysaccharid, vorzugsweise mindestens einen Desoxyzucker oder mindestens ein Desoxyzucker-Bausteine enthaltendes Polysaccharid.

Als Desoxyzucker im Sinne der Erfindung werden vorzugsweise die L(-)-Fucose und die L(+)-Rhamnose verstanden. Fucose kommt z.B. als Baustein von Polysacchariden vor, die aus marinen Braunalgen (z. B. Fucus vesiculosus) isoliert werden können, Rhamnose stellt einen Polysaccharid-Baustein der Arabinsäure in Gummi arabicum dar. Entsprechende Handelsprodukte sind z. B. Fucogel 1000 (INCI-Bezeichnung Biosaccharide Gum-1) oder Rhamnosoft (INCI-Bezeichnung Biosaccharide Gum-2), beide von der Firma Solabia erhältlich.

Besonders bevorzugte erfindungsgemäße Zusammensetzungen enthalten zusätzlich 0,5 bis 20 Gew.-% mindestens eines Polysaccharids, ausgewählt aus Polysacchariden, die mindestens einen Desoxyzucker-Baustein, insbesondere Fucose und/oder Rhamnose, enthalten, aus den Glycosaminoglycanen, besonders bevorzugt Hyaluronsäure, Dextran, Dextransulfat, Chondroitin-4-sulfat und Chondroitin-6-sulfat, weiterhin ausgewählt aus den Glucanen (Polyglucosanen), insbesondere Cellulose, Amylose, Glycogen, Lichenin, Laminarin aus Algen, Pachyman aus Baumpilzen und Hefeglucanen mit β-1,3-Bindung; Nigeran, und Pustulan (β-1,6-Glucan), Curdlan (β-1,3-D-Glucan), Pullulan (α-1,4-gebundenes und α-1,6-gebundenes D-Glucan) und Schizophyllan (β-1,3-Grund-kette, β-1,6-Seitenkette), weiterhin ausgewählt aus beta-(1,3-1,4)-Glucanen, chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat oder Dihydroxypropyldistärkephosphat, weiterhin Chitosan und dessen Salzen und anderen Derivaten, weiterhin aus Polysacchariden, die Gums oder Gummen bilden, wie Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Weitere erfindungsgemäß bevorzugte Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Das Glucose-Molekül kann α-glycosidisch oder β-glycosidisch verknüpft, unterschiedlich stark verzweigt oder linear angeordnet sein.

Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan) und Pustulan (β-1,6-Glucan).

Glucane sind unter anderem Bestandteil der Zellwand; sie werden von zahlreichen Mikroorganismen unter bestimmten physiologischen Bedingungen in ihr Milieu abgegeben oder auch erst dort synthetisiert. Dextran, ein primär α-1,6-gebundenes D-Glucan, ist das bedeutendste in dieser Polysaccharid-Gruppe. Weitere technisch interessante und erfindungsgemäß hervorragend geeignete Glucane sind Curdlan (β-1,3-D-Glucan), Pullulan (α-1,4-gebundenes und α-1,6-gebundenes D-Glucan) und Schizophyllan (β-1,3-Grundkette, β-1,6-Seitenkette). Glucane besitzen diverse immunmodulatorische Eigenschaften, auf Zelloberflächen des menschlichen Immunsystems befinden sich entsprechende Glucan-Rezeptoren.

Weitere erfindungsgemäß bevorzugte Polysaccharid-Wirkstoffe, deren Gegenwart eine unerwartete Wirkungssteigerung des mindestens einen Aporphin-Alkaloids der Formel (APO-ALK-1) bewirkt, sind ausgewählt aus beta-(1,3-1,4)-Glucanen. Besonders bevorzugt sind beta-(1,3-1,4)-Glucane, die zu etwa 70% beta-1,4-Glucosid-Verknüpfungen und zu etwa 30% beta-1,3-Glucosid-Verknüpfungen aufweisen. Derartige beta-(1,3-1,4)-Glucane sind bevorzugt erhältlich aus Extrakten aus Haferkörnern (Avena Sativa (Oat) Kernel Extract). Erfindungsgemäß besonders bevorzugte Extrakte aus Haferkörnern sind unter der Handelsbezeichnungen Drago Beta Glucan (02/060800) und SymGlucan von der Firma Symrise erhältlich.

Erfindungsgemäß ebenfalls bevorzugte Zusammensetzungen sind **dadurch gekennzeichnet, daß** sie 0,5 bis 20 Gew.-%, vorzugsweise 0,6 bis 15, weiter bevorzugt 0,7 bis 10, noch weiter bevorzugt 0,8 bis 7,5 und insbesondere 1 bis 5 Gew.-% beta-(1,3-1,4)-Glucane enthalten.

Besonders bevorzugt sind dabei solche Zusammensetzungen, die beta-(1,3-1,4)-Glucane enthalten, die zu 60-80% beta-1,4-Glucosid-Verknüpfungen und zu 20-40% beta-1,3-Glucosid-Verknüpfungen aufweisen.

Die erfindungsgemäßen Zusammensetzungen können im kosmetischen Träger niedere Alkohole enthalten, was im Hinblick auf die Konfektionierung des Produktes und einen zügig eintretenden Straffungseffekt bevorzugt ist. Insbesondere die physiologisch gut verträglichen niederen Alkohole Ethanol und Isopropanol führen in Verbindung mit der erfindungsgemäßen Polymerkombination zu Zusammensetzungen, die sich angenehm applizieren lassen, keine Fäden ziehen und eine sofortig wahrnehmbare Straffung der Haut bewirken. Erfindungsgemäß besonders bevorzugte Zusammensetzung enthalten daher 0,1 bis 10 Gew.-%, vorzugsweise 0,25 bis 7,5 Gew.-%, besonders bevorzugt 0,5 bis 5 Gew.-%, weiter bevorzugt 0,75 bis 4 Gew.-% und insbesondere 1 bis 3 Gew.-% Ethanol und/oder Isopropanol.

Im Folgenden werden bevorzugte Inhaltsstoffe der erfindungsgemäßen Mittel näher beschrieben.

Anionische Tenside sind bevorzugt in den erfindungsgemäßen Mitteln enthalten. Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, d.h. Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z.B. die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden insbesondere in Reinigungsmitteln aufgrund ihres hohen Schaumverhaltens vorzugsweise nur in relativ geringen Mengen, beispielsweise in Mengen von 1 bis 5 Gew.-%, eingesetzt. Vorzugsweise können die erfindungsgemäßen Mittel frei von Schwefelsäuremonoester sein.

Eine weitere Klasse von Aniontensiden ist die durch Umsetzung von Fettalkoholethoxylaten mit Natriumchloracetat in Gegenwart basischer Katalysatoren zugängliche Klasse der Ethercarbonsäuren. Sie haben die allgemeine Formel: R¹⁰ O-(CH₂-CH₂-O)p-CH₂-COOH mit R¹⁰ = C₁-C₁₈ und *p* = 0,1 bis 20. Ethercarbonsäuren sind wasserhärteunempfindlich und weisen ausgezeichnete Tensideigenschaften auf.

Geeignete anionische Tenside sind beispielsweise auch die Partialester von Di- oder Polyhydroxyalkanen, Mono- und Disacchariden, Polyethylenglycolen mit den EO-Addukten von Maleinsäureanhydrid an mindestens einfach ungesättigte Carbonsäuren mit einer Kettenlänge von 10 bis 25 Kohlenstoffatomen mit einer Säurezahl von 10 bis 140.

Bevorzugte anionische Tenside weisen neben einem unverzweigten oder verzweigten, gesättigten oder ungesättigten, aliphatischen oder aromatischen, acyclischen oder cyclischen, optional alkoxylierten Alkylrest mit 4 bis 28, vorzugsweise 6 bis 20, insbesondere 8 bis 18, besonders bevorzugt 10 bis 16, äußerst bevorzugt 12 bis 14 Kohlenstoffatomen, zwei oder mehr anionische, insbesondere zwei, Säuregruppen, vorzugsweise Carboxylat-, Sulfonat- und/oder Sulfatgruppen, insbesondere eine Carboxylat- und eine Sulfatgruppe, auf. Beispiele dieser Verbindungen sind die alpha-Sulfofettsäuresalze, die Acylglutamate, die Monoglyceriddisulfate und die Alkylether des Glycerindisulfats sowie insbesondere die nachfolgend beschriebenen monoveresterten Sulfosuccinate.

Besonders bevorzugte anionische Tenside sind die Sulfosuccinate, Sulfosuccinamate und Sulfosuccinamide, insbesondere Sulfosuccinate und Sulfosuccinamate, äußerst bevorzugt Sulfosuccinate. Bei den Sulfosuccinaten handelt es sich um die Salze der Mono- und Di-ester der Sulfobernsteinsäure HOOCCH(SO₃H)CH₂COOH, während man unter den Sulfosuccinamaten die Salze der Monoamide der Sulfobernsteinsäure und unter den Sulfosuccinamiden die Salze der Diamide der Sulfobernsteinsäure versteht.

Bei den Salzen handelt es sich bevorzugt um Alkalimetallsalze, Ammoniumsalze sowie Mono-, Di- bzw. Trialkanolammoniumsalze, beispielsweise Mono-, Di- bzw. Triethanolammoniumsalze, insbesondere um Lithium-, Natrium-, Kalium- oder Ammoniumsalze, besonders bevorzugt Natrium-oder Ammoniumsalze, äußerst bevorzugt Natriumsalze.

In den Sulfosuccinaten ist eine bzw. sind beide Carboxylgruppen der Sulfobernsteinsäure vorzugsweise mit einem bzw. zwei gleichen oder verschiedenen unverzweigten oder verzweigten, gesättigten oder ungesättigten, acyclischen oder cyclischen, optional alkoxylierten Alkoholen mit 4 bis 22, vorzugsweise 6 bis 20, insbesondere 8 bis 18, besonders bevorzugt 10 bis 16, äußerst bevorzugt 12 bis 14 Kohlenstoffatomen verestert. Besonders bevorzugt sind die Ester unverzweigter und/oder gesättigter und/oder acyclischer und/oder alkoxylierter Alkohole, insbesondere unverzweigter, gesättigter Fettalkohole und/oder unverzweigter, gesättigter, mit Ethylen- und/oder Propylenoxid, vorzugsweise Ethylenoxid, alkoxylierter Fettalkohole mit einem Alkoxylierungsgrad von 1 bis 20, vorzugsweise 1 bis 15, insbesondere 1 bis 10, besonders bevorzugt 1 bis 6, äußerst bevorzugt 1 bis 4. Die Monoester werden im Rahmen der vorliegenden Erfindung gegenüber den Diestern bevorzugt. Ein besonders bevorzugtes Sulfosuccinat ist Sulfobernsteinsäurelaurylpolyglycolester-di-Natrium-Salz (Lauryl-EO-sulfosuccinat, Di-Na-Salz; INCI Disodium Laureth Sulfosuccinate), das beispielsweise als Tego^{®} Sulfosuccinat F 30 (Goldschmidt) mit einem Sulfosuccinatgehalt von 30 Gew.-% kommerziell erhältlich ist.

In den Sulfosuccinamaten bzw. Sulfosuccinamiden bildet eine bzw. bilden beide Carboxylgruppen der Sulfobernsteinsäure vorzugsweise mit einem primären oder sekundären Amin, das einen oder zwei gleiche oder verschiedene, unverzweigte oder verzweigte, gesättigte oder ungesättigte, acyclische oder cyclische, optional alkoxylierte Alkylreste mit 4 bis 22, vorzugsweise 6 bis 20, insbesondere 8 bis 18, besonders bevorzugt 10 bis 16, äußerst bevorzugt 12 bis 14 Kohlenstoffatomen trägt, ein Carbonsäureamid. Besonders bevorzugt sind unverzweigte und/oder gesättigte und/oder acyclische Alkylreste, insbesondere unverzweigte, gesättigte Fettalkylreste.

Weiterhin geeignet sind beispielsweise die folgenden gemäß INCI bezeichneten Sulfosuccinate und Sulfosuccinamate, die im *International Cosmetic Ingredient Dictionary and Handbook* näher beschrieben sind: Ammonium Dinonyl Sulfosuccinate, Ammonium Lauryl Sulfosuccinate, Diammonium Dimethicone Copolyol Sulfosuccinate, Diammonium Lauramido-MEA Sulfosuccinate, Diammonium Lauryl Sulfosuccinate, Diammonium Oleamido PEG-2 Sulfosuccinate, Diamyl Sodium Sulfosuccinate, Dicapryl Sodium Sulfosuccinate, Dicyclohexyl Sodium Sulfosuccinate, Diheptyl Sodium Sulfosuccinate, Dihexyl Sodium Sulfosuccinate, Diisobutyl Sodium Sulfosuccinate, Dioctyl Sodium Sulfosuccinate, Disodium Cetearyl Sulfosuccinate, Disodium Cocamido MEA-Sulfosuccinate, Disodium CocamidoGlucoside Sulfosuccinate, Disodium Cocoyl Butyl Gluceth-10 Sulfosuccinate, Disodium C12-15 Pareth Sulfosuccinate, Disodium Deceth-5 Sulfosuccinate, Disodium Deceth-6 Sulfosuccinate, Disodium Dihydroxyethyl Sulfosuccinylundecylenate, Disodium Dimethicone Copolyol Sulfosuccinate, Disodium Hydrogenated Cottonseed Glyceride Sulfosuccinate, Disodium Isodecyl Sulfosuccinate, Disodium Isostearamido MEA-Sulfosuccinate, Disodium Isostearamido MIPA-Sulfosuccinate, Disodium Isostearyl Sulfosuccinate, Disodium Laneth-5 Sulfosuccinate, Disodium Lauramido MEA-Sulfosuccinate, Disodium Lauramido PEG-2 Sulfosuccinate, Disodium Lauramido PEG-5 Sulfosuccinate, Disodium Laureth-6 Sulfosuccinate, Disodium Laureth-9 Sulfosuccinate, Disodium Laureth-12 Sulfosuccinate, Disodium Lauryl Sulfosuccinate, Disodium Myristamido MEA-Sulfosuccinate, Disodium Nonoxynol-10 Sulfosuccinate, Disodium Oleamido MEA-Sulfosuccinate, Disodium Oleamido MIPA-Sulfosuccinate, Disodium Oleamido PEG-2 Sulfosuccinate, Disodium Oleth-3 Sulfosuccinate, Disodium Oleyl Sulfosuccinate, Disodium Palmitamido PEG-2 Sulfosuccinate, Disodium Palmitoleamido PEG-2 Sulfosuccinate, Disodium PEG-4 Cocamido MIPA-Sulfosuccinate, Disodium PEG-5 Laurylcitrate Sulfosuccinate, Disodium PEG-8 Palm Glycerides Sulfosuccinate, Disodium Ricinoleamido MEA-Sulfosuccinate, Disodium Sitostereth-14 Sulfosuccinate, Disodium Stearamido MEA-Sulfosuccinate, Disodium Stearyl Sulfosuccinamate, Disodium Stearyl Sulfosuccinate, Disodium Tallamido MEA-Sulfosuccinate, Disodium Tallowamido MEA-Sulfosuccinate, Disodium Tallow Sulfosuccinamate, Disodium Tridecylsulfosuccinate, Disodium Undecylenamido MEA-Sulfosuccinate, Disodium Undecylenamido PEG-2 Sulfosuccinate, Disodium Wheat Germamido MEA-Sulfosuccinate, Disodium Wheat Germamido PEG-2 Sulfosuccinate, Di-TEA-Oleamido PEG-2 Sulfosuccinate, Ditridecyl Sodium Sulfosuccinate, Sodium Bisglycol Ricinosulfosuccinate, Sodium/MEA Laureth-2 Sulfosuccinate und Tetrasodium Dicarboxyethyl Stearyl Sulfosuccinamate. Noch ein weiteres geeignetes Sulfosuccinamat ist Dinatrium-C₁₆₋₁₈-alkoxypropylensulfosuccinamat.

Eine weitere geeignete Verbindungsklasse sind Aniontensid-Kation-Komplexe, wobei das Kation selbst ursprünglich tensidische Eigenschaften aufweist. Beispiele sind Umesterungsprodukte von LAS-Säure mit Aminen, Aminderivaten, enthaltend eine C-Kette mit > 2 C-Atomen, vorzugsweise C₁₂-C₁₆. Diese können z.B. am Stickstoff oxidiert sein, d.h. z.B. Umesterung von LAS-Säure mit Aminoxid C₁₂₋₁₄.

Der Gehalt des erfindungsgemäßen Mittels an anionischen Tensiden, vorzugsweise an den genannten anionischen Tensiden, kann in weiten Bereichen variieren, je nachdem welchem Zweck das betreffende Mittel dient. So kann ein erfindungsgemäßes Mittel sehr große Mengen Aniontensid enthalten, vorzugsweise bis zu einer Größenordnung von bis zu 40, 50 oder 60 Gew.-oder mehr. Ebenso kann ein erfindungsgemäßes Mittel nur sehr geringe Mengen Aniontensid enthalten, beispielsweise weniger als 15 oder 10 Gew.-% oder weniger als 5 Gew.-% oder noch weniger. Vorteilhafterweise sind in den erfindungsgemäßen Mitteln jedoch Aniontenside in Mengen von 0,1 bis 40 Gew.-% und insbesondere 5 bis 30 Gew.-% enthalten, wobei Konzentrationen oberhalb von 10 Gew.-% und sogar oberhalb von 15 Gew.-% besondere Bevorzugung finden. Nach einer bevorzugten Ausführungsform enthält das erfindungsgemäße Mittel anionische Tenside, vorzugsweise in Mengen von zumindest 0,01 Gew.-%, bezogen auf das gesamte Mittel. Nach einer anderen bevorzugten Ausführungsform kann das erfindungsgemäße Mittel frei von Aniontensid sein.

Zusätzlich zu den genannten anionischen Tensiden, aber auch unabhängig von diesen, können in den erfindungsgemäßen Mitteln Seifen enthalten sein. Geeignet sind insbesondere gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische. Der Gehalt des Mittels an Seifen beträgt, unabhängig von anderen Aniontensiden, vorzugsweise nicht mehr als 3 Gew.-% und insbesondere 0,5 bis 2,5 Gew.-%, bezogen auf das gesamte Mittel. Nach einer anderen bevorzugten Ausführungsform ist das erfindungsgemäße Mittel frei von Seife.

Die anionischen Tenside und Seifen können in Form ihrer Natrium-, Kalium- oder Ammoniumsalze sowie als lösliche Salze organischer Basen, wie Mono-, Di- oder Triethanol-amin, vorliegen. Vorzugsweise liegen sie in Form ihrer Natrium- oder Kaliumsalze, insbesondere in Form der Natriumsalze vor. Anionische Tenside und Seifen können auch in situ hergestellt werden, indem in die sprühzutrocknende Zusammensetzung die Aniontensidsäuren und gegebenenfalls Fettsäuren eingebracht werden, welche dann durch die Alkaliträger in der sprühzutrocknenden Zusammensetzung neutralisiert werden.

Vorteilhafterweise können nichtionische Tenside in den erfindungsgemäßen Mitteln ebenfalls enthalten sein, sowohl in festen wie in flüssigen Mitteln. Beispielsweise kann ihr Gehalt bis zu 2 oder 3 oder 5 Gew.-% betragen. Es können auch größere Mengen an nichtionischem Tensid enthalten sein, beispielsweise bis zu 5 Gew.-% oder 10 Gew.-% oder 15 Gew.-% oder 20 Gew.-%, 30 Gew.-%, 40 Gew.-%, 50 Gew.-% oder sogar darüber hinaus, falls es zweckmäßig ist. Sinnvolle Untergrenzen können bei Werten von 0,01, 0,1, 1, 2, 3 oder 4 Gew.-% liegen.

Vorzugsweise sind die nichtionischen Tenside aber in größeren Mengen, also bis zu 50 Gew.-%, vorteilhafterweise von 0,1 bis 40 Gew.-%, besonders bevorzugt von 0,5 bis 30 und insbesondere von 2 bis 25 Gew.-%, jeweils bezogen auf das gesamte Mittel, enthalten. Nach einer bevorzugten Ausführungsform enthält das erfindungsgemäße Mittel nichtionische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Niotensid sein.

Vorteilhafterweise können alle aus dem Stand der Technik bekannten nichtionischen Tenside in den erfindungsgemäßen Mitteln enthalten sein. Bevorzugte Niotenside werden weiter unten vorgestellt.

Die erfindungsgemäßen Hautbehandlungsmittel können vorzugsweise auch mindestens ein kationisches Tensid enthalten. Geeignete Kationtenside sind beispielsweise oberflächenaktive quaternäre Verbindungen, insbesondere mit einer Ammonium-, Sulfonium-, Phosphonium-, Jodonium- oder Arsoniumgruppe. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

Besonders bevorzugte kationische Tenside sind die quaternären, z.T. antimikrobiell wirkenden Ammoniumverbindungen (QAV; INCI Quaternary Ammonium Compounds) gemäß der allgemeinen Formel (R^{I})(R^{II})(R^{III})(R^{IV})N⁺ X⁻, in der R^{I} bis R^{IV} gleiche oder verschiedene C₁₋₂₂-Alkylreste_{'} C₇₋₂₈-Aralkyl-reste oder heterozyklische Reste, wobei zwei oder im Falle einer aromatischen Einbindung wie im Pyridin sogar drei Reste gemeinsam mit dem Stickstoffatom den Heterozyklus, z.B. eine Pyridinium- oder Imidazoliniumverbindung, bilden, darstellen und X⁻ Halogenidionen, Sulfationen, Hydroxidionen oder ähnliche Anionen sind. Für eine optimale antimikrobielle Wirkung weist vorzugsweise wenigstens einer der Reste eine Kettenlänge von 8 bis 18, insbesondere12 bis 16, C-Atomen auf.

QAV sind durch Umsetzung tertiärer Amine mit Alkylierungsmitteln, wie z.B. Methylchlorid, Benzylchlorid, Dimethylsulfat, Dodecylbromid, aber auch Ethylenoxid herstellbar. Die Alkylierung von tertiären Aminen mit einem langen Alkyl-Rest und zwei Methyl-Gruppen gelingt besonders leicht, auch die Quaternierung von tertiären Aminen mit zwei langen Resten und einer MethylGruppe kann mit Hilfe von Methylchlorid unter milden Bedingungen durchgeführt werden. Amine, die über drei lange Alkyl-Reste oder Hydroxy-substituierte Alkyl-Reste verfügen, sind wenig reaktiv und werden bevorzugt mit Dimethylsulfat quaterniert.

Geeignete QAV sind beispielsweise Benzalkoniumchlorid (N-Alkyl-N,N-dimethyl-benzylammonium-chlorid, CAS No. 8001-54-5), Benzalkon B (m,p-Dichlorbenzyl-dimethyl-C₁₂-alkylammoniumchlorid, CAS No. 58390-78-6), Benzoxoniumchlorid (Benzyl-dodecyl-bis-(2-hydroxyethyl)-ammoniumchlorid), Cetrimoniumbromid (N-Hexadecyl-N,N-trimethyl-ammoniumbromid, CAS No. 57-09-0), Benzetoniumchlorid (N,N-Dimethyl-N-[2-[2-[p-(1,1,3,3-tetramethylbutyl)phenoxy]ethoxy]ethyl]-benzylammo-niumchlorid, CAS No. 121-54-0), Dialkyldimethylammoniumchloride wie Di-n-decyldimethyl-ammo-niumchlorid (CAS No. 7173-51-5-5), Didecyldimethylammoniumbromid (CAS No. 2390-68-3), Dioctyl-dimethyl-ammoniumchloric, 1-Cetylpyridiniumchlorid (CAS No. 123-03-5) und Thiazolinjodid (CAS No. 15764-48-1) sowie deren Mischungen. Bevorzugte QAV sind die Benzalkoniumchloride mit C₈-C₁₈-Alkylresten, insbesondere C₁₂-C₁₄-Aklyl-benzyl-dimethylammoniumchlorid. Eine besonders bevorzugte QAV Kokospentaethoxymethylammoniummethosulfat *(INCI* PEG-5 Cocomonium Methosulfate; Rewoquat^{®} CPEM).

Zur Vermeidung möglicher Inkompatibilitäten der antimikrobiellen kationischen Tenside mit in dem erfindungsgemäßen Mittel enthaltenen anionischen Tensiden werden möglichst aniontensidverträgliches und/oder ggf. möglichst wenig kationisches Tensid eingesetzt oder in einer besonderen Ausführungsform der Erfindung gänzlich auf kationische Tenside verzichtet.

Weiter unten werden insbesondere im Zusammenhang mit Konditioniermitteln und Weichmachern weitere kationische Tenside, so auch quartäre Ammoniumverbindungen beschrieben. Auch diese können vorzugsweise in den erfindungsgemäßen Mitteln enthalten sein.

Die erfindungsgemäßen Hautbehandlungsmittel können ein oder mehrere kationische Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Geeignete Mindestwerte können auch bei 0,5, 1, 2 oder 3 Gew.-% liegen. Nach einer bevorzugten Ausführungsform enthält das erfindungsgemäße Mittel kationische Tenside, vorzugsweise in Mengen von zumindest 0,1 Gew.-%, bezogen auf das gesamte Mittel. Nach einer anderen bevorzugten Ausführungsform kann das erfindungsgemäße Mittel frei von Kationtensid sein.

Ebenso können die erfindungsgemäßen Hautbehandlungsmittel auch mindestens ein amphoteres Tensid enthalten. Diese werden weiter unten insbesondere im Zusammenhang mit Konditioniermitteln und Weichmachern noch näher beschrieben.

Die erfindungsgemäßen Hautbehandlungsmittel können ein oder mehrere amphotere Tenside enthalten, vorteilhafterweise in Mengen, bezogen auf die Gesamtzusammensetzung, von 0 bis 30 Gew.-%, noch vorteilhafter größer 0 bis 20 Gew.-%, vorzugsweise 0,01 bis 10 Gew.-%, insbesondere 0,1 bis 5 Gew.-%. Nach einer anderen bevorzugten Ausführungsform kann das erfindungsgemäße Mittel frei von amphoteren Tensiden sein.

Nach einer bestimmten Ausführungsform können die erfindungsgemäßen Mittel nur sehr wenig Gesamttensid enthalten, z.B. kann die Gesamttensidmenge unter 20 Gew.-%, 15 Gew.-%, 10 Gew.-% oder 5 Gew.-%, vorteilhafterweise sogar unter 3 Gew.-% oder unter 1 Gew.-%, insbesondere sogar unter 0,5 Gew.-% oder unter 0,1 Gew.-% liegen, Gew.-% jeweils bezogen auf das gesamte Mittel. Vorzugsweise beträgt der Gesamttensidgehalt aber zumindest 0,01 Gew.-%, 0,1 Gew-% oder 1 Gew.-%, bezogen auf das gesamte Mittel.

Lösliche Komplexbildner kann das erfindungsgemäße Mittel vorzugsweise in Mengen von 0,1 Gew.-% bis 30 Gew.-%, bevorzugt 5 Gew.-% bis 25 Gew.-% und besonders bevorzugt 10 Gew.-% bis 20 Gew.-%, bezogen auf das Gesamtgewicht des Mittel, enthalten, wobei Ethylendiamintetraeesigsäure und ihre Natriumsalze als lösliche Komplexbildner besonders bevorzugt sind. Es kann aber vorteilhafterweise auch vorgesehen sein, dass das erfindungsgemäße Mittel weniger als 10 Gew.-%, beispielsweise weniger als 5 Gew.-% lösliche Komplexbildner enthält. Geeignet sind beispielsweise Citrate, SKS-6, Citronensäure, MGDA (methyl glycine di-acetic acid), Triphosphate, Phosphonate, aliphatische Dicarbonsäuren (z.B. Adipin-, Glutar-, Bernsteinsäure).

Nach einer anderen bevorzugten Ausführungsform kann das erfindungsgemäße Mittel frei von löslichen Komplexbildnern sein.

Weitere geeignete organische Komplexbildner sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500000 g/mol. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch so genannte Gelbdextrine und Weißdextrine mit höheren Molmassen im Bereich von 2000 bis 30000 g/mol. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

Auch Oxydisuccinate und andere Derivate von Disuccinaten, vorzugsweise Ethylendiamindisuccinat, sind weitere geeignete Cobuilder. Dabei wird Ethylendiamin-N,N'-di-succinat (EDDS) bevorzugt in Form seiner Natrium- oder Magnesiumsalze verwendet. Weiterhin bevorzugt sind in diesem Zusammenhang auch Glycerindisuccinate und Glycerintrisuccinate. Geeignete Einsatzmengen liegen beispielsweise bei 3 bis 15 Gew.-%, bezogen auf das gesamte Mittel.

Weitere brauchbare organische Co-Komplexbildner sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

Eine weitere Substanzklasse mit Co-Komplexbildner-Eigenschaften stellen die Phosphonate dar. Dabei handelt es sich insbesondere um Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Co-Komplexbildner. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden.

Bevorzugte organische Komplexbildner sind beispielsweise die in Form ihrer Alkali- und insbesondere Natriumsalze einsetzbaren Polycarbonsäuren, wie Citronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Citronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer komplexierenden Wirkung typischerweise auch die Eigenschaft einer Säuerungskomponente und dienen somit auch zur Einstellung eines niedrigeren und milderen pH-Wertes bevorzugter erfindungsgemäßer Hautbehandlungsmittel. Insbesondere sind hierbei Citronensäure, Bernsteinsäure, Glutarsäure, Adipinsäure, Gluconsäure und beliebige Mischungen aus diesen zu nennen.

Komplexbildner (INCI Chelating Agents), auch Sequestriermittel genannt, sind Inhaltsstoffe, die Metallionen zu komplexieren und inaktivieren vermögen, beispielsweise um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert die oxidative Zersetzung der fertigen Mittel.

Geeignet sind beispielsweise die folgenden gemäß INCI bezeichneten Komplexbildner, die beispielsweise im International Cosmetic Ingredient Dictionary and Handbook näher beschrieben sind: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphos-phate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Bevorzugte Komplexbildner sind tertiäre Amine, insbesondere tertiäre Alkanolamine (Aminoalkohole). Die Alkanolamine besitzen sowohl Amino- als auch Hydroxy- und/oder Ether-gruppen als funktionelle Gruppen. Besonders bevorzugte tertiäre Alkanolamine sind Tri-ethanolamin und Tetra-2-hydroxypro-pylethylendiamin (N,N,N',N'-Tetrakis-(2-hydroxy-pro-pyl)ethylendiamin). Besonders bevorzugte Kombinationen tertiärer Amine mit Zinkricinoleat und einem oder mehreren ethoxylierten Fettalkoholen als nichtionische Lösungsvermittler sowie ggf. Lösungsmittel sind im Stand der Technik beschrieben.

Ein besonders bevorzugter Komplexbildner ist die Etidronsäure (1-Hydroxyethyliden-1,1-diphosphon-säure, 1-Hydroxyethyan-1,1-diphosphonsäure, HEDP, Acetophosphonsäure, INCI Etidronic Acid) einschließlich ihrer Salze. In einer bevorzugten Ausführungsform enthält das erfindungsgemäße Mittel demgemäß als Komplexbildner Etidronsäure und/oder eines oder mehrere ihrer Salze.

In einer besonders bevorzugten Ausführungsform enthält das erfindungsgemäße Mittel eine Komplexbildnerkombination aus einem oder mehreren tertiären Aminen und einer oder mehreren weiteren Komplexbildnern, vorzugsweise einer oder mehreren Komplexbildnersäuren oder deren Salzen, insbesondere aus Triethanolamin und/oder Tetra-2-hydroxypropylethylendiamin und Etidronsäure und/oder einem oder mehrerer ihrer Salze.

Das erfindungsgemäße Mittel enthält bevorzugt mindestens einen Komplexbildner in einer Gesamtmenge von üblicherweise 0 bis 20 Gew.-%, vorzugsweise 0,1 bis 15 Gew.-%, insbesondere 0,5 bis 10 Gew.-%, besonders bevorzugt 1 bis 8 Gew.-%, äußerst bevorzugt 1,5 bis 6 Gew.-%, bezogen auf das gesamte Mittel.

Es sei an dieser Stelle darauf hingewiesen, dass sich die Angabe Gew.-%, sofern es nicht anders angegeben ist, jeweils auf das gesamte Mittel bezieht.

Der Gehalt an Wasser in bevorzugten erfindungsgemäßen Mitteln richtet sich u.a. danach, ob das Mittel in flüssiger oder fester Form vorliegt, beträgt daher vorzugsweise 0 bis weniger als 100 Gew.-% und insbesondere 0,5 bis 95 Gew.-%, wobei Werte von maximal 5 Gew.-% insbesondere bei festen oder nichtwässrigen flüssigen Mitteln besondere Bevorzugung finden. Nicht miteingerechnet wurde hierbei das in einzelnen Rohstoffen, wie insbesondere den schweißhemmenden Aluminium- und/ode Zirconium-Verbindungen, vorhandene Kristallwasser.

Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.-%., in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel.

Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% Gew.-% liegen, bezogen auf das gesamte Mittel.

Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel.

Die genannten Ober- und Untergrenzen können natürlich sinnvoll kombiniert werden, z.B. zu Wassergehalten von 60-80 Gew.-% oder 10-30 Gew.-% usw.

In einer weiteren bevorzugten Ausführungsform enthält das erfindungsgemäße Hautbehandlungsmittel zusätzlich mindestens eine UV-absorbierende Substanz. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzy-liden)campher; 4-Aminobenzoesäure-derivate, vorzugsweise 4-(Di-methylamino)benzoesäure-2-ethylhexylester, 4-(Dimethylamino)benzoe-säure-2-octylester und 4-(Dimethylamino)benzoe-säureamylester; Ester der Zimtsäure, vorzugsweise 4-Meth-oxyzimtsäure-2-ethylhexylester, 4-Methoxyzimtsäurepropylester, 4-Methoxyzimtsäureiso-amyl-ester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4'-methylbenzophenon, 2,2'-Dihy-droxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo-(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zink-stearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten. Diese Mengen schließen das mindestens eine erfindungsgemäß verwendete photokatalytisch aktive Metalloxid, das ebenfalls im UV-Bereich absorbieren kann, nicht ein.

In einer weiteren bevorzugten Ausführungsform liegen die erfindungsgemäßen Mittel in flüssiger Form vor. Zum Erreichen einer flüssigen Konsistenz kann der Einsatz sowohl flüssiger organischer Lösungsmittel, wie auch der von Wasser angezeigt sein. Bevorzugte erfindungsgemäße Mittel enthalten daher gegebenenfalls mindestens ein Lösungsmittel.

Lösungsmittel, die in bevorzugten erfindungsgemäßen Mitteln eingesetzt werden können, stammen beispielsweise aus der Gruppe ein- oder mehrwertigen Alkohole, Alkanolamine oder Glycolether, sofern sie im angegebenen Konzentrationsbereich mit Wasser mischbar sind. Vorzugsweise werden die Lösungsmittel ausgewählt aus Ethanol, n- oder i-Propanol, Butanolen, Glycol, Propan- oder Butandiol, Glycerin, Diglyol, Propyl- oder Butyldiglycol, Hexylenglycol, Ethylenglycolmethylether, Ethylenglycolethylether, Ethylen-glycolpropylether, Ethylenglycolmono-n-butylether, Diethylenglycol-methylether, Diethylenglycolethylether, Propylenglycolmethyl-, -ethyl- oder -propyl-ether, Butoxy-propoxy-propanol (BPP), Dipropylenglycolmonomethyl-, oder - ethylether, Di-isopropylenglycol-monomethyl-, oder -ethylether, Methoxy-, Ethoxy- oder Butoxytriglycol, 1-Butoxyethoxy-2-propanol, 3-Methyl-3-methoxybutanol, Propylen-glycolt-butylether sowie Mischungen dieser Lösungsmittel.

Einige Glycolether sind unter den Handelsnamen Arcosolv^{®} (Arco Chemical Co.) oder Cellosolve^{®}, Carbitol^{®} oder Propasol^{®} (Union Carbide Corp.) erhältlich; dazu gehören auch z.B. ButyICarbitol^{®}, HexyICarbitol^{®}, MethyICarbitol^{®}, und Carbitol^{®} selbst, (2-(2-Ethoxy)ethoxy)ethanol. Die Wahl des Glycolethers kann vom Fachmann leicht auf der Basis seiner Flüchtigkeit, Wasserlöslichkeit, seines Gewichtsprozentanteils an der gesamten Dispersion und dergleichen getroffen werden. Pyrrolidon-Lösungsmittel, wie N-Alkyl-pyrrolidone, beispielsweise N-Methyl-2-pyrrolidon oder N-C₈-C₁₂-Alkyl-pyrrolidon, oder 2-Pyrrolidon, können ebenfalls eingesetzt werden. Weiterhin bevorzugt als alleinige Lösungsmittel oder als Bestandteil eines Lösungsmittelgemisches sind Glycerinderivate, insbesondere Glycerincarbonat.

Zu den Alkoholen, die in der vorliegenden Erfindung vorzugsweise als Cosolventien eingesetzt werden können, gehören flüssige Polyethylenglycole, mit niederem Molekulargewicht, beispielsweise Polyethylenglycole mit einem Molekulargewicht von 200, 300, 400 oder 600. Weitere geeignete Cosolventien sind andere Alkohole, zum Beispiel (a) niedere Alkohole wie Ethanol, Propanol, Isopropanol und n-Butanol, (b) Ketone wie Aceton und Methylethylketon, (c) C₂-C₄-Polyole wie ein Diol oder ein Triol, beispielsweise Ethylenglycol, Propylenglycol, Glycerin oder Gemische davon. Insbesondere bevorzugt ist aus der Klasse der Diole 1,2-Octandiol.

In einer bevorzugten Ausführungsform enthält das erfindungsgemäße Mittel ein oder mehrere Lösungsmittel aus der Gruppe, umfassend C₁- bis C₄-Monoalko-hole, C₂- bis C₆-Glycole, C₃- bis C₁₂-Glycolether und Glycerin, insbesondere Ethanol. Die erfindungsgemäßen C₃- bis C₁₂-Glycolether enthalten Alkyl- bzw. Alkenylgruppen mit weniger als 10 Kohlenstoffatomen, vorzugsweise bis zu 8, insbesondere bis zu 6, besonders bevorzugt 1 bis 4 und äußerst bevorzugt 2 bis 3 Kohlenstoffatomen.

Bevorzugte C₁- bis C₄-Monoalkohole sind Ethanol, n-Propanol, iso-Propanol und tert-Butanol. Bevorzugte C₂- bis C₆-Glycole sind Ethylenglycol, 1,2-Propylenglycol, 1,3-Propylenglycol, 1,5-Pentandiol, Neopentylglycol und 1,6-Hexandiol, insbesondere Ethylenglycol und 1,2-Propylenglycol. Bevorzugte C₃- bis C₁₂-Glycolether sind Di-, Tri-, Tetra- und Pentaethylenglycol, Di-, Tri-und Tetrapropylenglycol, Propylenglycolmonotertiärbutylether und Propylenglycolmonoethylether sowie die gemäß INCI bezeichneten Lösungsmittel Butoxydiglycol, Butoxyethanol, Butoxyisopropanol, Butoxypropanol, Butyloctanol, Ethoxydiglycol, Ethoxyethanol, Ethyl Hexanediol, Isobutoxypropanol, Isopentyldiol, 3-Methoxybutanol, Methoxyethanol, Methoxyisopropanol und Methoxymethylbutanol.

Bevorzugte erfindungsgemäße Mittel enthalten ein oder mehrere Lösungsmittel in einer Gesamtmenge von üblicherweise bis zu 90 Gew.-%, vorzugsweise 0,1 bis 30 Gew.-%, insbesondere 2 bis 20 Gew.-%, besonders bevorzugt 3 bis 15 Gew.-%, äußerst bevorzugt 5 bis 12 Gew.-%, beispielsweise 5,3 oder 10,6 Gew.-%, jeweils bezogen auf das gesamte Mittel.

Farbstoffe können optional im erfindungsgemäßen Mittel eingesetzt werden, wobei die Menge an einem oder mehreren Farbstoffen so gering zu wählen ist, dass nach der Anwendung des Mittels keine sichtbaren Rückstände verbleiben. Vorzugsweise ist das erfindungsgemäße Mittel frei von Farbstoffen.

Das erfindungsgemäße Mittel kann vorzugsweise einen oder mehrere antimikrobielle Wirkstoffe bzw. Konservierungsmittel in einer Menge von üblicherweise 0,0001 bis 3 Gew.-%, vorzugsweise 0,0001 bis 2 Gew.-%, insbesondere 0,0002 bis 1 Gew.-%, besonders bevorzugt 0,0002 bis 0,2 Gew.-%, äußerst bevorzugt 0,0003 bis 0,1 Gew.-%, enthalten.

Antimikrobielle Wirkstoffe bzw. Konservierungsmittel unterscheidet man je nach antimikrobiellem Spektrum und Wirkungsmechanismus zwischen Bakteriostatika und Bakteriziden, Fungistatika und Fungiziden usw. Wichtige Stoffe aus diesen Gruppen sind beispielsweise Benzalkoniumchloride, Alkylarylsulfonate, Halogenphenole und Phenolmercuriacetat. Die Begriffe antimikrobielle Wirkung und antimikrobieller Wirkstoff haben im Rahmen der erfindungsgemäßen Lehre die fachübliche Bedeutung. Geeignete antimikrobielle Wirkstoffe sind vorzugsweise ausgewählt aus den Gruppen der Alkohole, Amine, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-acetale sowie -formale, Benzamidine, Isothiazoline, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-di-cyanobutan, lodo-2-propyl-butyl-carbamat, lod, lodophore, Peroxoverbindungen, Halogenverbindungen sowie beliebigen Gemischen der voranstehenden.

Der antimikrobielle Wirkstoff kann dabei ausgewählt sein aus Ethanol, n-Propanol, i-Pro-panol, 1,3-Butandiol, Phenoxyethanol, 1,2-Propylenglycol, Glycerin, Undecylensäure, Benzoesäure, Salicylsäure, Dihydracetsäure, o-Phenylphenol, N-Methylmorpholin-aceto-nitril (MMA), 2-Benzyl-4-chlorphenol, I, 2,2'-Methylen-bis-(6-brom-4-chlorphenol), 4,4'-Di-chlor-2'-hydroxydiphenylether (Dichlosan), 2,4,4'-Trichlor-2'-hydroxydiphenylether (Trichlosan), Chlorhexidin, N-(4-Chlorphenyl)-N-(3,4-dichlorphenyl)-harnstoff, N,N'-(1,10-decan-diyldi-1-pyridinyl-4-yliden)-bis-(1-octanamin)-dihydrochlorid, N,N'-Bis-(4-chlorphenyl)-3,12-dümino-2,4,11,13-tetraaza-tetradecandümidamid, Glucoprotaminen, antimikrobiellen oberflächenaktiven quaternären Verbindungen, Guanidinen einschl. den Bi- und Polyguani-dinen, wie beispielsweise 1,6-Bis-(2-ethylhexyl-biguanido-hexan)-dihydrochlorid, 1 ,6-Di-(N₁,N₁'-phenyldiguanido-N₅,N₅')-hexan-tetrahydochlorid, 1,6-Di-(N_{1,}N₁'-phenyi-N₁,N₁-methyidiguanido-N₅,N₅')-hexan-dihydro-chiorid, 1,6-Di-(N₁,N₁'-o-chloro-phenyldiguanido- N₅,N_{5'})-hexan-dihydrochlorid, 1,6-Di-(N₁,N₁'-2,6-dichlorophenyldiguanido-N₅,N₅')-hexan-dihydrochlorid, 1,6-Di-[N₁,N₁'-beta-(p-methoxyphenyl) diguanido-N₅,N₅']-hexane-dihydrochlorid, 1,6-Di-(N₁,N₁'-aipha-methyi-.beta.-phenyidiguanido-N₅,N₅')-hexan-dihydro-chiorid, 1,6-Di-(N₁,N₁'-p-nitrophenyldiguanido-N₅,N₅')hexan-dihydrochlorid, omega:omega-Di-( N₁,N₁'-phenyldiguanido-N,₅,N,₅')-di-n-propylether-dihydrochlorid, omega:omega'-Di-(N₁,N₁'-p-chlorophenyldiguanido-N₅,N₅')-di-n-propylether-tetrahydrochlorid, 1,6-Di-(N₁,N₁'-2,4-dichlorophenyldiguanido-N₅,N₅')hexan-tetrahydrochlorid, 1,6-Di-(N₁,N₁'-p-methylphe-nyldiguanido-N₅,N₅')hexan-dihydrochlorid, 1,6-Di-(N₁,N₁'-2,4,5-trichiorophenyidi-guanido-N₅,N₅')hexan-tetrahydrochiorid, 1,6-Di-[N₁,N₁'-alpha-(p-chlorophenyl) ethyldiguanido-N₅,N₅'] hexan-dihydrochlorid, omega:omega-Di-(N₁,N₁'-p-chlorophe-nyldiguanido-N₅,N₅')m-xylene-dihydrochlorid, 1,12-Di-(N₁,N₁'-p-chlorophenyldiguanido-N₅,N₅') dodecan-dihydro-chlorid, 1,10-Di-(N₁,N₁'-phenyldiguanido-N₅,N₅')-decan-tetrahydrochlorid, 1,12-Di-(N₁,N₁'-phenyidiguanido- N₅,N₅') dodecan-tetrahydrochlorid, 1,6-Di-(N₁,N₁'-o-chlorophenyldi-guanido- N₅,N₅') hexan-dihydrochlorid, 1,6-Di-(N₁,N₁'-o-chlorophenyl-diguanido- N₅,N₅') hexan-tetrahydrochlorid, Ethylen-bis-(1-tolyl biguanid), Ethylen-bis-(p-tolyl biguanide), Ethylen-bis-(3,5-dimethylphenylbiguanid), Ethylen-bis-(p-tert-amylphenylbiguanid), Ethylen-bis-(nonylphenylbiguanid), Ethylen-bis-(phenylbi-guanid), Ethylen-bis-(N-butylphenylbiguanid), Ethylen-bis (2,5-diethoxyphenylbiguanid), Ethylen-bis (2,4-dimethylphenyl biguanid), Ethylen-bis (o-diphenylbiguanid), Ethylen-bis (mixed amyl naphthylbiguanid), N-Butyl-ethylen-bis-(phenylbiguanid), Trimethylenbis(o-tolylbiguanid), N-Butyl-trimethyle-bis-(phenyl biguanide) und die entsprechenden Salze wie Acetate, Gluconate, Hydrochloride, Hydrobromide, Citrate, Bisulfite, Fluoride, Polymaleate, N-Cocosalkylsarcosinate, Phosphite, Hypophosphite, Perfluorooctanoate, Silicate, Sorbate, Salicylate, Maleate, Tartrate, Fumarate, Ethylendiamintetraacetate, Iminodiacetate, Cinnamate, Thiocyanate, Arginate, Pyromellitate, Tetracarboxybutyrate, Benzoate, Glutarate, Monofluorphosphate, Perfluorpropionate sowie beliebige Mischungen davon. Weiterhin eignen sich halogenierte Xylol- und Kresolderivate, wie p-Chlormetakresol oder p-Chlormetaxylol, sowie natürliche antimikrobielle Wirkstoffe pflanzlicher Herkunft (z.B. aus Gewürzen oder Kräutern), tierischer sowie mikrobieller Herkunft. Vorzugsweise können antimikrobiell wirkende oberflächenaktive quaternäre Verbindungen, ein natürlicher antimikrobieller Wirkstoff pflanzlicher Herkunft und/oder ein natürlicher antimikrobieller Wirkstoff tierischer Herkunft, äußerst bevorzugt mindestens ein natürlicher antimikrobieller Wirkstoff pflanzlicher Herkunft aus der Gruppe, umfassend Coffein, Theobromin und Theophyllin sowie etherische Öle wie Eugenol, Thymol und Geraniol, und/ oder mindestens ein natürlicher antimikrobieller Wirkstoff tierischer Herkunft aus der Gruppe, umfassend Enzyme wie Eiweiß aus Milch, Lysozym und Lactoperoxidase, und/ oder mindestens eine antimikrobiell wirkende oberflächenaktive quaternäre Verbindung mit einer Ammonium-, Sulfonium-, Phosphonium-, lodonium- oder Arsoniumgruppe, Peroxoverbindungen und Chlorverbindungen eingesetzt werden. Auch Stoffe mikrobieller Herkunft, sogenannte Bakteriozine, können eingesetzt werden. Bevorzugt werden Glycin, Glycinderivate, Formaldehyd, Verbindungen, die leicht Formaldehyd abspalten, Ameisensäure und Peroxide verwendet.

Die als antimikrobielle Wirkstoffe geeigneten quaternären Ammoniumverbindungen (QAV) sind oben schon beschrieben worden. Besonders geeignet ist beispielsweise Benzalkoniumchlorid etc. Benzalkoniumhalogenide und/ oder substituierte Benzalkoniumhalogenide sind beispielsweise kommerziell erhältlich als Barquat^{®} ex Lonza, Marquat^{®} ex Mason, Variquat^{®} ex Witco/ Sherex und Hyamine^{®} ex Lonza, sowie Bardac^{®} ex Lonza. Weitere kommerziell erhältliche antimikrobielle Wirkstoffe sind N-(3-Chlorallyl)-hexaminiumchlorid wie Dowicide^{®} und Dowicil^{®} ex Dow, Benzethoniumchlorid wie Hyamine^{®} 1622 ex Rohm & Haas, Methylbenzethoniumchlorid wie Hyamine^{®} 10X ex Rohm & Haas, Cetylpyridiniumchlorid wie Cepacolchlorid ex Merrell Labs.

Weitere bevorzugte erfindungsgemäße Zusammensetzungen sind **dadurch gekennzeichnet, dass** zur Verbesserung der Hautfeuchtigkeit unter Verzicht auf komedogene Substanzen mindestens ein alkyl- oder hydroxyalkylsubstituierten Harnstoff der allgemeinen Formel (A) enthalten ist, 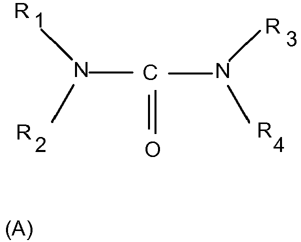 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (A) ausgewählt ist aus Bis-N,N'- (2-hydroxyethyl)harnstoff.

Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance der National Starch and Chemical Company als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻) jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind **dadurch gekennzeichnet, dass** sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methyl-bernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind **dadurch gekennzeichnet, dass** sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-a-L-mannopyranosyl)-β-D-glucopyranosid)), Monoxerutin (3,3',4',5-Tetrahydroxy-7-(2-hydroxy-ethoxy)-flavon-3-(6-O-(6-deoxy-a-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Erfindungsgemäß besonders bevorzugte Zusammensetzungen sind **dadurch gekennzeichnet, dass** sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Fam-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättem von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kemen der Chardonnay-Traube, erhältlich von der Firma Croda.

Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Erfindungsgemäß besonders bevorzugte Zusammensetzungen sind **dadurch gekennzeichnet, dass** sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1 ,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.

Erfindungsgemäß besonders bevorzugte Zusammensetzungen sind **dadurch gekennzeichnet, dass** sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten.

Olivenblattextrakte können einen hohen Gehalt an Oleanolsäure und/oder Oleanol aufweisen. In einer weiteren bevorzugten Ausführungsform enthalten die erfindungsgemäßen Zusammensetzungen Oleanolsäure, Oleanol und/oder Oleuropein. Erfindungsgemäß besonders bevorzugte Zusammensetzungen sind **dadurch gekennzeichnet, dass** sie Oleanolsäure, Oleanol und/oder Oleuropein in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind **dadurch gekennzeichnet, dass** sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind **dadurch gekennzeichnet, dass** sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C® , Exsymol).

Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung.

In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind **dadurch gekennzeichnet, dass** sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens einen Extrakt aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Dieser Extrakt erhöht und/oder verbessert die Interaktion zwischen der extrazellulären Matrix und den Fibroblasten.

Erfindungsgemäß besonders bevorzugte kosmetische oder dermatologische Zusammensetzungen sind **dadurch gekennzeichnet, dass** sie mindestens einen Wirkstoff, der ausgewählt ist aus Extrakten aus Maiskörnern (Zea Mays (Corn) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind **dadurch gekennzeichnet, dass** sie mindestens einen Wirkstoff, der ausgewählt ist aus Extrakten aus Maiskörnern (Zea Mays (Corn) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich. Dieser Extrakt erhöht und/oder verbessert die Interaktion zwischen der extrazellulären Matrix und den Fibroblasten. Erfindungsgemäß besonders bevorzugte kosmetische oder dermatologische Zusammensetzungen sind **dadurch gekennzeichnet, dass** sie mindestens einen Wirkstoff, der ausgewählt ist aus Extrakten aus Haferkörnern (Avena Sativa (Oat) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind **dadurch gekennzeichnet, dass** sie mindestens einen Wirkstoff, der ausgewählt ist aus Extrakten aus Haferkörnern (Avena Sativa (Oat) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Produkt, das durch Fermentation von gezuckertem Schwarztee mit den zwei symbiotischen Mikroorganismen Saccharomyces und Acetobacter ylinum gewonnen wird, als Kombucha bezeichnet wird und die INCI-Bezeichnung Saccharomyces/ Xylinum/Black Tea Ferment trägt. Derartige Produkte erhöhen und/oder verbessern die Interaktion zwischen der extrazellulären Matrix und den Fibroblasten. Ein besonders bevorzugtes Produkt ist unter dem Handelsnamen Kombuchka von der Firma Sederma erhältlich (INCI-Bezeichnung: Saccharomyces/Xylinum/Black Tea Ferment, Glycerin, Hydroxyethylcellulose).

Erfindungsgemäß besonders bevorzugte kosmetische oder dermatologische Zusammensetzungen sind **dadurch gekennzeichnet, dass** sie mindestens einen Wirkstoff, der ausgewählt ist aus Produkten, die durch Fermentation von gezuckertem Schwarztee mit den zwei symbiotischen Mikroorganismen Saccharomyces und Xylinum gewonnen werden und die INCI-Bezeichnung Saccharomyces/Xylinum/ Black Tea Ferment tragen, in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Produkt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Acetobacter Xylinum gewonnen werden und die INCI-Bezeichnung Saccharomyces/Xylinum/Black Tea Ferment tragen, in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Apfelkernextrakten (Pyrus Malus (Apple) Fruit Extract). Derartige Extrakte erhöhen und/oder verbessern die Interaktion zwischen der extrazellulären Matrix und den Fibroblasten. Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.

Erfindungsgemäß besonders bevorzugte kosmetische oder dermatologische Zusammensetzungen sind **dadurch gekennzeichnet, dass** sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Lotuskeimen (Nelumbo Nucifera Germ Extract). Derartige Extrakte erhöhen und/oder verbessern die Interaktion zwischen der extrazellulären Matrix und den Fibroblasten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.

Erfindungsgemäß besonders bevorzugte kosmetische oder dermatologische Zusammensetzungen sind **dadurch gekennzeichnet, dass** sie mindestens einen Extrakt aus Lotuskeimen (Nelumbo Nucifera Germ Extract) in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Extrakt aus Lotuskeimen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Rotwein. Derartige Extrakte erhöhen und/ oder verbessern die Interaktion zwischen der extrazellulären Matrix und den Fibroblasten. Ein erfindungsgemäß besonders bevorzugter Rotweinextrakt ist unter dem Handelsnamen Sepivinol R von der Firma Seppic erhältlich.

Erfindungsgemäß besonders bevorzugte kosmetische oder dermatologische Zusammensetzungen sind **dadurch gekennzeichnet, dass** sie mindestens einen Extrakt aus Rotwein in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Extrakt aus Rotwein in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Traubenkernen (Vitis Vinifera (Grape) Seed Extract). Derartige Extrakte erhöhen und/oder verbessern die Interaktion zwischen der extrazellulären Matrix und den Fibroblasten. Besonders bevorzugt stammen die Traubenkernextrakte aus der Chardonnay-Traube. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.

Erfindungsgemäß besonders bevorzugte kosmetische oder dermatologische Zusammensetzungen sind **dadurch gekennzeichnet, dass** sie mindestens einen Extrakt aus Traubenkernen in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Extrakt aus Traubenkernen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Schwarzen Holunderblüten (Sambucus Nigra Flower Extract). Derartige Extrakte erhöhen und/oder verbessern die Interaktion zwischen der extrazellulären Matrix und den Fibroblasten. Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.

Erfindungsgemäß besonders bevorzugte kosmetische oder dermatologische Zusammensetzungen sind **dadurch gekennzeichnet, dass** sie mindestens einen Extrakt aus Schwarzen Holunderblüten in Mengen von 0,1 - 10 Gew.-%, bevorzugt 1 - 5 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind **dadurch gekennzeichnet, dass** sie mindestens einen Extrakt aus Schwarzen Holunderblüten in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Wirkstoff, der die beta-Endorphinsynthese in Keratinozyten stimuliert. Erfindungsgemäß besonders bevorzugte Stimulatoren der beta-Endorphin-Synthese sind ausgewählt aus Mischungen aus mindestens einem Extrakt aus den Blättern der Mentha piperita und mindestens einem Extrakt aus Kakaobohnen, wobei wässrige, glycolische oder wässrig-glycolische Zubereitungen dieser Extraktmischungen, die unter den Handelsbezeichnungen Caomint, Caophenol, Caobromine, Caospice und Caoorange von der Firma Solabia erhältlich sind, besonders bevorzugt sind. Ein weiterer besonders bevorzugter Stimulator der beta-Endorphin-Synthese ist das Dipeptidderivat N-Acetyl-Tyr-Arg-hexyldecylester mit der INCI-Bezeichnung Acetyl Dipeptide-1 Cetyl Ester, das z. B. als wässrige Zubereitung unter der Handelsbezeichnung Calmosensine von der Firma Sederma erhältlich ist.

Weitere bevorzugte Stimulatoren der beta-Endorphin-Synthese sind Extrakte aus Helichrysum italicum, z. B. erhältlich unter der Handelsbezeichnung Areaumat Perpetua von der Firma Codif, Extrakte aus Crithmum Maritimum, z. B. erhältlich unter den Handelsbezeichnungen Areaumat Samphira und Aroleat Samphira von der Firma Codif, Extrakte aus Lavendula stoechas, z. B. erhältlich unter der Handelsbezeichnung Areaumat Lavanda von der Firma Codif, Extrakte aus Mentha piperita, wie sie z. B. unter den Handelsbezeichnungen Authenticals of Peppermint (Solabia) und Calmiskin (Silab) erhältlich sind, Glutamylamidoethyl Indole, z. B. erhältlich unter der Handelsbezeichnung Glistin von der Firma Exsymol, ein durch mikrobielle Fermentation gewonnenes verzweigtes Polysaccharid mit Rhamnose-, Galactose- und Glucuronsäure-Einheiten mit der INCI-Bezeichnung Biosaccharide Gum-2, z. B. erhältlich unter der Handelsbezeichnung Rhamnosoft von der Firma Solabia, Extrakte aus den Samen von Tephrosia Purpurea mit der INCI-Bezeichnung Tephrosia Purpurea Seed Extract, z. B. erhältlich unter der Handelsbezeichnung Tephroline von der Firma Vincience, Mischungen aus dem Öl von Mentha arvensis-Blättern, Limonenschalenöl, Zypressenöl, Lavendelöl und Cistus Ladaniferus-Öl mit der INCI-Bezeichnung Mentha Arvensis Leaf Oil and Citrus Medica Limonum (Lemon) Peel Oil and Cupressus Sempervirens Oil and Lavandula Hybrida Oil and Cistus Ladaniferus Oil, z. B. erhältlich unter der Handelsbezeichnung V-Tonic (Gattefosse), und Hexasaccharide gemäß FR 2842201 sowie beliebige Mischungen dieser Wirkstoffe.

Erfindungsgemäß besonders bevorzugte kosmetische oder dermatologische Zusammensetzungen sind **dadurch gekennzeichnet, dass** sie mindestens einen Wirkstoff zur Stimulation der beta-Endorphin-Synthese in Gesamtmengen von 0,01 - 10 Gew.-%, bevorzugt 0,1 - 5 Gew.-% und besonders bevorzugt 1 - 3 Gew.-%, jeweils bezogen auf das Handelsprodukt, das den Wirkstoff enthält, in der gesamten erfindungsgemäßen Zusammensetzung, enthalten. Erfindungsgemäß besonders bevorzugte kosmetische oder dermatologische Zusammensetzungen sind **dadurch gekennzeichnet, dass** sie mindestens einen Wirkstoff zur Stimulation der beta-Endorphin-Synthese in Gesamtmengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten erfindungsgemäßen Zusammensetzung, enthalten.

In einer weiteren bevorzugten Ausführungsform enthalten die erfindungsgemäßen Zusammensetzungen zur Unterstützung der Wirkung des erfindungsgemäß verwendeten photokatalytisch aktiven Metalloxids, insbesondere Titandioxid, mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria, Glycyrrhizin, das auch als Glycyrrhizinsäure oder Glycyrrhetinsäureglycosid bezeichnet wird und das 2-beta-Glucuronido-alpha-glucuronid der Glycyrrhetinsäure darstellt, sowie deren Salzen, Gerbsäure (tannic acid) und deren Salzen, Gallotanninen, Naringin, Mischungen aus Glycyrrhizin-(salzen), Gerbsäure(salzen) und/oder Gallotanninen und Naringin, wie sie zum Beispiel als Handelsprodukt BiSCos Glynarin PF (INCI: Aqua (Water), Alacohol, Ohenoxyethanol, Ammonium Glycyrrhizate, Tannic Acid, Naringine) von der Firma Biesterfeld erhältlich sind, weiterhin aus wasser- und öllöslichen Extrakten aus Hamamelis, Klettenwurzel und Brennessel, Zimtbaumextract (z. B. Sepicontrol® A5 von der Firma Seppic), Chrysanthemenextrakt (z. B. Laricyl® von Laboratoires Sérobiologiques), Hefeproteinhydrolysaten, wie sie z.B. in den Handelsprodukten der Asebiol®-Serie von Laboratoires Sérobiologiques erhältlich sind, insbesondere Asebiol^{®} LS 2539 BT 2 (INCI: Aqua, Hydrolyzed Yeast Protein, Pyridoxine, Niacinamide, Glycerin, Panthenol, Allantoin, Biotin) und Asebiol^{®} LS 2539 BT (Aqua, Hydrolyzed Yeast Protein, Pyridoxine, Niacinamide, Glycerin, Panthenol, Propylene Glycol, Allantoin, Disodium Azelate, Biotin) und PEG-8 Isolauryl Thioether, wie es z. B. in dem Handelsprodukt "Antifettfaktor^{®} COS-218/2-A" von Cosmetochem enthalten ist (INCI: Aqua, Cetyl-PCA, PEG-8 Isolauryl Thioether, PCA, Cetyl Alcohol), sowie Mischungen der vorgenannten Substanzen. Bevorzugte Mischungen sind beispielsweise erhältlich als Handelsprodukt Acnacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind **dadurch gekennzeichnet, dass** sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Zur Unterstützung des erfindungsgemäß verwendeten photokatalytisch aktiven Metalloxids, insbesondere Titandioxid, enthalten die erfindungsgemäßen Hautbehandlungsmittel bevorzugt weiterhin mindestens einen partikelförmigen, sebumsorbierenden Wirkstoff, insbesondere Talkum, Stärke und andere Partikel.

Erfindungsgemäß bevorzugte kosmetische oder dermatologische Zusammensetzungen sind dadurch gekennzeichnet, dass der partikelförmige, sebumsorbierende Wirkstoff ausgewählt ist aus anorganischen und organischen kosmetischen Adsorbentien mit mittleren Partikeldurchmessern von 0,1 - 100 µm, bevorzugt 0,5 - 50 µm, besonders bevorzugt 5 - 30 µm und außerordentlich bevorzugt 10 - 25 µm. Besonders bevorzugte anorganische Adsorbentien sind ausgewählt aus Kieselsäuren, insbesondere Aerosil^{®}-Typen, Kieselgelen, Siliciumdioxid, Tonen und Schichtsilicaten, insbesondere Bentoniten oder Kaolin, Magnesiumaluminiumsilikaten, insbesondere Talkum, und Bornitrid, sowie Mischungen der genannten Substanzen. Besonders bevorzugte organische Adsorbentien sind ausgewählt aus Stärken und Stärkederivaten, die chemisch und/oder physikalisch modifiziert sein können, insbesondere modifizierten Stärkederivaten vom Typ DRY FLO^{®} der National Starch and Chemical Company, Cellulosepulvern, Lactoglobulinderivaten, insbesondere Natrium-C₈₋₁₆-Isoalkylsuccinyllactoglobulinsulfonat, z. B. als Handelsprodukt Biopol^{®} OE von Brooks Industries erhältlich, Polymerpulvern aus Polyolefinen, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, wobei die genannten Polymerpulver in einer besonders bevorzugten Ausführungsform der Erfindung vernetzt sein können, sowie Mischungen der genannten Substanzen.

Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind vernetzte Polymethacrylate (Micropearl^{®} M von SEPPIC oder Plastic Powder A von NIKKOL), vernetzte Polymethylmethacrylate (Micropearl^{®} M 305 und Micropearl^{®} M 310 von SEPPIC), Styrol-Divinylbenzol-Copolymere (z. B. Plastic Powder FP von NIKKOL), Polyethylen- und Polypropylen-Pulver (z. B. ACCUREL^{®} EP 400 von AKZO) oder auch Siliconpolymere (z. B. Silicone Powder X2-1605 von Dow Corning).

In einer weiteren bevorzugten Ausführungsform der Erfindung weist der partikelförmige, sebumsorbierende Wirkstoff Hohlräume auf. In einer weiteren bevorzugten Ausführungsform der Erfindung weist der partikelförmige, sebumsorbierende Wirkstoff halbkugelförmige Partikel auf, besonders bevorzugt sind mindestens 50 Gew.-% der Partikel halbkugelförmig. In einer weiteren bevorzugten Ausführungsform der Erfindung weist der partikelförmige, sebumsorbierende Wirkstoff halbkugelförmige Partikel mit Hohlräumen auf, besonders bevorzugt sind mindestens 50 Gew.-% der Partikel halbkugelförmig mit Hohlräumen.

Besonders bevorzugte partikelförmige, sebumsorbierende Wirkstoffe sind vernetzte Polymethylmethacrylate mit mittleren Partikeldurchmessern von 5 - 50 µm, insbesondere die Handelsprodukte Micropearl^{®} M 305 und Micropearl^{®} M 310 von SEPPIC. Außerordentlich bevorzugte partikelförmige, sebumsorbierende Wirkstoffe sind vernetzte Polymethylmethacrylate mit mittleren Partikeldurchmessern von 5 - 50 µm, die halbkugelförmige Partikel mit Hohlräumen umfassen, wobei besonders bevorzugt mindestens 50 Gew.-% der Partikel halbkugelförmig mit Hohlräumen sind, wie insbesondere das Handelsprodukt Micropearl^{®} M 310 von SEPPIC.

Erfindungsgemäß besonders bevorzugte Zusammensetzungen sind **dadurch gekennzeichnet, dass** sie mindestens einen partikelförmigen sebumsorbierenden Wirkstoff in einer Gesamtmenge von 0,5 bis 10 Gew.-%, bevorzugt 1 bis 5 Gew.-% und besonders bevorzugt 1,5 bis 3 Gew.-%, jeweils bezogen auf die gesamte Zusammensetzung, enthalten.

In einer weiteren bevorzugten Ausführungsform enthalten die erfindungsgemäßen Zusammensetzungen mindestens einen organischen hautaufhellenden Wirkstoff. Erfindungsgemäß bevorzugte hautaufhellende Wirkstoffe sind einzeln oder in Mischungen ausgewählt aus Hydrochinon, Kojisäure, Arbutin, Extrakten aus Süßholzwurzel (Glycyrrhiza glabra), Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Ascorbinsäure, Cumarinsäure ((Z)-2-Hydroxyzimtsäure) und cis-9-Octadecendisäure (andere Nomenklatur: cis-8-Hexadecendicarbonsäure), letztere kommerziell erhältlich z. B. unter der Bezeichnung Arlatone DIOIC DCA von Uniqema, und den Estern und/oder Salzen dieser Säuren. Erfindungsgemäß besonders bevorzugte kosmetische oder dermatologische Zusammensetzungen sind **dadurch gekennzeichnet, dass** sie mindestens einen organischen hautaufhellenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die sogenannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Mit dem Begriff Parfümöl sind vorzugsweise in sich abgeschlossene Duftstoffkompositionen gemeint, die gemeinhin zur Produktbeduftung eingesetzt werden und insbesondere nach menschlichem Ermessen wohlriechend sind. Dies sei an einem Beispiel erläutert. Will ein Fachmann z.B. ein Deodorant wohlriechend machen, so fügt er ihm für gewöhnlich nicht nur eine (wohl-)riechende Substanz, sondern ein Kollektiv (wohl-)riechender Substanzen bei. Ein solches Kollektiv besteht gewöhnlich aus einer Vielzahl einzelner Riechstoffe, z.B. mehr als 10 oder 15, vorzugsweise bis zu 100 oder mehr. Diese Riechstoffe formen zusammenwirkend ein gewünschtes wohlriechendes, harmonisches Geruchsbild.

Ein erfindungsgemäßes Parfümöl kann einzelne Riechstoffverbindungen, z.B. die synthetischen Produkte vom Typ der Ester, Ether, Aldehyde, Ketone, Alkohole und Kohlenwasserstoffe enthalten. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexyl-propionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote des gebildeten Parfümöl erzeugen.

Die Parfümöle können aber auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" (top note), "Herz- bzw. Mittelnote" (middle note bzw. body) sowie "Basisnote" (end note bzw. dry out) unterteilt.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, a-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphthol-methylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümöl vorteilhaft einsetzbar sind, zählen insbesondere die niedriger siedenden Riechstoffe natürlichen oder synthetischen Usprung, die allein oder in Mischungen eingesetzt werden können. Beispiele für leichter flüchtige Riechstoffe sind Alkyisothiocyanate (Alkylsenföle), Butandion, Limonen, Linalool, Linalylacetat und -propionat, Menthol, Menthon, Methyl-n-hep-tenon, Phellandren, Phenylacetaldehyd, Terpinylacetat, Zitral, Zitronellal.

Alle vorgenannten Riechstoffe sind alleine oder in Mischung in den Parfümölen gemäß der vorliegenden Erfindung mit den bereits genannten Vorteilen einsetzbar.

Insbesondere können auch Duftsstoffe aus der Gruppe der Allylalkoholester, Ester sekundärer Alkohole, Ester tertiärer Alkohole, allylische Ketone, Acetale, Ketale, Kondensationsprodukte von Aminen und Aldehyden und/oder deren Mischungen im Parfümöl enthalten sein.

In einer bevorzugten Ausführungsform enthält das erfindungsgemäße Mittel bestimmte Minimalwerte an Parfümöl, nämlich zumindest 0,00001 Gew.-%, vorteilhafterweise zumindest 0,0001 Gew.-%, in beträchtlich vorteilhafter Weise zumindest 0,001 Gew.-%, in vorteilhafterer Weise zumindest 0,01 Gew.-%, in weiter vorteilhafter Weise zumindest 0,1 Gew.-%, in noch weiter vorteilhafter Weise zumindest 0,2 Gew.-%, in sehr vorteilhafter Weise zumindest 0,3 Gew.-%, in besonders vorteilhafter Weise zumindest 0,4 Gew.-%, in ganz besonders vorteilhafter Weise zumindest 0,45 Gew.-%, in erheblich vorteilhafter Weise zumindest 0,5 Gew.-%, in ganz erheblich vorteilhafter Weise zumindest 0,55 Gew.-%, in äußerst vorteilhafter Weise zumindest 0,6 Gew.-%, in höchst vorteilhafterweise zumindest 0,65 Gew.-%, in überaus vorteilhafterweise zumindest 0,7 Gew.-%, in ausnehmend vorteilhafter Weise zumindest 0,75 Gew.-%, in außergewöhnlich vorteilhafter Weise zumindest 0,8 Gew.-%, in außerordentlich vorteilhafter Weise zumindest 0,85 Gew.-%, insbesondere zumindest 0,9 Gew.-% an Parfümöl, bezogen auf das gesamte Mittel.

In einer bevorzugten Ausführungsform enthalten die Parfümöle weniger als 8, vorteilhafterweise weniger als 7, in vorteilhafterer Weise weniger als 6, in wiederum vorteilhafterer Weise weniger als 5, in weiter vorteilhafterweise weniger als 4, noch vorteilhafter weniger als 3, vorzugsweise weniger als 2, insbesondere keine Duftstoffe aus der Liste Amylcinnamal, Amylcinnamylalkohol, Benzylalkohol, Benzylsalicylat, Cinnamylalkohol, Cinnamal, Citral, Cumarin, Eugenol, Geraniol, Hydroxycitronellal, Hydroxymethylpentylcyclohexencarboxaldehyd, Isoeugenol, Anisylalkohol, Benzylbenzoat, Benzylcinnamat, Citronellol, Farnesol, Hexylcinnamaldehyd, Lilial, d-Limonen, Linalool, Methylheptincarbonat, 3-Methyl-4-(2,6,6-trimethyl-2-cyclohexen-1-yl)-3-buten-2-on, Eichenmoosextrakt, Baummoosextrakt.

Nach einer weiteren bevorzugten Ausführungsform ist das erfindungsgemäße Mittel frei von Parfümöl-Komponenten, insbesondere in Produkten für sensitive Haut.

Ein weiterer Gegenstand der vorliegenden Erfindung ist die Nicht-therapeutische, kosmetische Verwendung einer erfindungsgemäßen kosmetischen oder dermatologischen Zusammensetzung zur Anti-Cellulite-Behandlung der Haut und/oder zur Steigerung der Hautfestigkeit und/oder zur Verbesserung der mechanischen Stabilität der Haut und/oder zur Steigerung der Hautelastizität und/oder der Hautstraffheit und/oder zur Glättung der Haut und/oder zur Verbesserung des optischen Erscheinungsbildes der Haut und/oder zur Anregung der Mikrozirkulation in der Haut und/oder zur Unterstützung der Lipolyse in der Subcutis und/oder zur Inhibierung der Lipogenese in der Subcutis.

Bezüglich weiterer bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, dadurch gekennzeichnet, daß ein erfindungsgemäßes Hautbehandlungsmittel auf die Haut aufgetragen wird.

Auch bezüglich weiterer bevorzugter Ausführungsformen der erfindungsgemäßen Verfahren gilt mutatis mutandis das zu den erfindungsgemäßen Mitteln Gesagte.

## Patentansprüche

1. Kosmetische oder dermatologische Zusammensetzung zur topischen Behandlung der Haut, enthaltend in einem geeigneten kosmetischen oder dermatologischen Träger - jeweils bezogen auf ihr Gewicht -
a) 0,01 bis 5 Gew.-% Xanthan,
b) 0,01 bis 5 Gew.-% Dehydroxanthan,
c) 0,1 bis 10 Gew.-% Polystyrolsulfonat(e).

2. Zusammensetzung nach Anspruch 1, **dadurch gekennzeichnet, daß** sie 0,05 bis 4 Gew.-%, vorzugsweise 0,1 bis 3 Gew.-%, weiter bevorzugt 0,2 bis 2 Gew.-%, noch weiter bevorzugt 0,25 bis 1,5 Gew.-% und insbesondere 0,3 bis 1 Gew.-% Xanthan enthält.

3. Zusammensetzung nach einem der Ansprüche 1 oder 2, **dadurch gekennzeichnet, daß** sie 0,05 bis 4 Gew.-%, vorzugsweise 0,1 bis 3 Gew.-%, weiter bevorzugt 0,2 bis 2 Gew.-%, noch weiter bevorzugt 0,25 bis 1,5 Gew.-% und insbesondere 0,4 bis 1 Gew.-% Dehydroxanthan enthält.

4. Zusammensetzung nach einem der Ansprüche 1 bis 3, **dadurch gekennzeichnet, daß** sie 0,25 bis 7,5 Gew.-%, vorzugsweise 0,5 bis 5 Gew.-%, weiter bevorzugt 0,75 bis 4 Gew.-%, noch weiter bevorzugt 1 bis 3 Gew.-% und insbesondere 1,5 bis 2,5 Gew.-% Polystyrolsulfonat(e) enthält.

5. Zusammensetzung nach einem der Ansprüche 1 bis 4, **dadurch gekennzeichnet, daß** sie 0,25 bis 7,5 Gew.-%, vorzugsweise 0,5 bis 5 Gew.-%, weiter bevorzugt 0,75 bis 4 Gew.-%, noch weiter bevorzugt 1 bis 3 Gew.-% und insbesondere 1,5 bis 2,5 Gew.-% Natrium-Polystyrolsulfonat 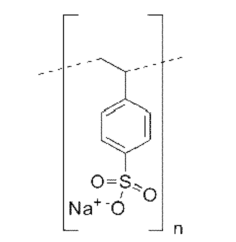 mit n = 100 bis 1000, vorzugsweise 200 bis 900, weiter bevorzugt 300 bis 800, noch weiter bevorzugt 400 bis 750 und insbesondere 600 bis 700, enthält.

6. Zusammensetzung nach einem der Ansprüche 1 bis 5, **dadurch gekennzeichnet, daß** sie zusätzlich 0,5 bis 20 Gew.-% mindestens eines Polysaccharids, ausgewählt aus Polysacchariden, die mindestens einen Desoxyzucker-Baustein, insbesondere Fucose und/oder Rhamnose, enthalten, aus den Glycosaminoglycanen, besonders bevorzugt Hyaluronsäure, Dextran, Dextransulfat, Chondroitin-4-sulfat und Chondroitin-6-sulfat, weiterhin ausgewählt aus den Glucanen (Polyglucosanen), insbesondere Cellulose, Amylose, Glycogen, Lichenin, Laminarin aus Algen, Pachyman aus Baumpilzen und Hefeglucanen mit β-1,3-Bindung; Nigeran, und Pustulan (β-1,6-Glucan), Curdlan (β-1,3-D-Glucan), Pullulan (α-1,4-gebundenes und α-1,6-gebundenes D-Glucan) und Schizophyllan (β-1,3-Grundkette, β-1,6-Seitenkette), weiterhin ausgewählt aus beta-(1,3-1,4)-Glucanen, chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat oder Dihydroxypropyldistärkephosphat, weiterhin Chitosan und dessen Salzen und anderen Derivaten, weiterhin aus Polysacchariden, die Gums oder Gummen bilden, wie Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar, enthält.

7. Zusammensetzung nach einem der Ansprüche 1 bis 6, **dadurch gekennzeichnet, daß** sie 0,5 bis 20 Gew.-%, vorzugsweise 0,6 bis 15, weiter bevorzugt 0,7 bis 10, noch weiter bevorzugt 0,8 bis 7,5 und insbesondere 1 bis 5 Gew.-% beta-(1,3-1,4)-Glucane enthält.

8. Zusammensetzung nach Anspruch 7, **dadurch gekennzeichnet, daß** sie beta-(1,3-1,4)-Glucane enthält, die zu 60-80% beta-1,4-Glucosid-Verknüpfungen und zu 20-40% beta-1,3-Glucosid-Verknüpfungen aufweisen.

9. Zusammensetzung nach einem der Ansprüche 1 bis 8, **dadurch gekennzeichnet, daß** sie 0,1 bis 10 Gew.-%, vorzugsweise 0,25 bis 7,5 Gew.-%, besonders bevorzugt 0,5 bis 5 Gew.-%, weiter bevorzugt 0,75 bis 4 Gew.-% und insbesondere 1 bis 3 Gew.-% Ethanol und/oder Isopropanol enthält.

10. Nicht-therapeutische, kosmetische Verwendung einer kosmetischen oder dermatologischen Zusammensetzung gemäß einem der Ansprüche 1 bis 9 zur Anti-Cellulite-Behandlung der Haut und/oder zur Steigerung der Hautfestigkeit und/oder zur Verbesserung der mechanischen Stabilität der Haut und/oder zur Steigerung der Hautelastizität und/oder der Hautstraffheit und/oder zur Glättung der Haut und/oder zur Verbesserung des optischen Erscheinungsbildes der Haut und/oder zur Anregung der Mikrozirkulation in der Haut und/oder zur Unterstützung der Lipolyse in der Subcutis und/oder zur Inhibierung der Lipogenese in der Subcutis.
